# EUROPEAN PATENT APPLICATION

(11) **EP 4 474 813 A1**
(43) Date of publication of application: **11.12.2024**
(21) Application number: 23315238.8
(22) Date of filing: 05.06.2023
(51) Int. Cl.: G01N 33/00, G06N 3/08

(54) **HANDLING OF CHEMICAL FAMILIES FOR AN ELECTRONIC NOSE**

(71) Applicant: Aryballe, 38000 Grenoble (FR)
(72) Inventor: Singh, Krishna Kant, 38000 Grenoble (FR)
(74) Representative: Schuffenecker, Thierry

(57) **Abstract**

There is disclosed a system comprising: an electronic nose, configured to sense one or more Volatile Organic Compounds in gas phase; one or more machine-learned models configured to receive data as input from said electronic nose and to output multi-class chemical families; wherein one or more machine-learned models have been trained using a labeled training set comprising signatures and annotated chemical families and/or comprising sensorgrams. Developments describe the use of low-dimensional embedding in a chemical space; mapping to another embedding space such as an odor-space; classification; determining vapor pressure, hydrophobicity and/or chemical properties; the use of various sensors including one or more of a humidity sensor, a temperature sensor, a photoionized sensor, or a gas-specific sensor; training by supervised learning and/or semi-supervised learning and/or metric learning and/or online-learning and/or incremental learning and/or transfer learning; the use of attention-based models; implementations occurring inline or in-device, or via remotely accessed computing resources.

## Description

### Technical field

The described examples are related to computer systems based on specific computational models and investigating or analyzing materials by determining their chemical or physical properties and recognition of data; presentation of data; record carriers; handling record carriers and electric digital data processing and image data processing or generation, in general.

### Background Art

Identifying chemical families from an electronic nose (e-nose) is a valuable application in various fields such as food quality control, environmental monitoring, and medical diagnostics. Moreover, identification of chemical families could be useful to gain insights into the aroma characteristics, quality, authenticity, and potential sensory impacts of substances.

Different chemical families tend to exhibit specific odor profiles due to the presence of certain functional groups or molecular configurations. For example, aldehydes often have a strong, pungent odor, while esters are associated with fruity or floral scents. Similarly, sulfur compounds are notorious for their characteristic rotten egg or skunk-like odors. Odor perception is a result of complex interactions between multiple volatile compounds present in a substance. The presence and relative concentrations of various compounds within a chemical family can influence the overall odor perception. For instance, the combination of different esters, alcohols, and aldehydes contributes to the distinctive aroma of fruits..

Identifying chemical families in the gas phase is a complex task due to several factors. Gas mixtures can contain a wide range of compounds from different chemical families, leading to overlapping signals and difficulties in distinguishing between specific families.

E-noses typically consist of arrays of sensors that respond to different volatile compounds. However, the responses of these sensors can overlap, meaning that multiple sensors may respond to the same chemical family or compounds with similar properties. This overlap can be challenging to differentiate between different chemical families. Additionally, e-noses often have limited selectivity, meaning that they can detect a broad range of compounds but may not be able to discriminate between similar compounds or different chemical families with similar sensor responses. Furthermore, e-nose sensors can experience drift over time, leading to changes in their responses even in the absence of chemical stimuli. Sensor aging can also occur, affecting their sensitivity and selectivity.

The proposed invention overcomes these challenges by integration of machine learning with electronic noses data to address the complexities and challenges associated with chemical family identification in the gas phase. These techniques enhance the selectivity, accuracy, and robustness of e-nose systems, enabling more reliable and efficient applications in areas such as food quality control, environmental monitoring, and medical diagnostics.

An "odor" (American English) or "odour" (British English) is caused by Volatile Organic Compounds (VOCs) and/or molecules emitted by objects in the environment, which can be found in low concentrations in the air that humans and animals can perceive by their sense of smell. Other terms with different connotations can be used e.g., "smell", "scent", "aroma", "fragrance", "perfumes", etc.

In nature, there are millions of volatile organic compounds. Generating their odor profile with an electronic nose is very challenging. Moreover, generating odor profiles of mixtures of VOCs is even more challenging.

Today, known methods use molecular modeling and deep learning-based approaches to build predictive models that consider signatures of pure VOCs. These methods work fine for liquid phases, but not for gas phases (for which problems are significantly more difficult to handle)

The article entitled "SMILE to smell" was published on January 15, 2021 in J. Chem. Inf. Model. 2021, 61, 2, 676-688 converts SMILES (simplified molecular-input line-entry system values) into images and uses CNN networks. According to the article, finding the relationship between the structure of an odorant molecule and its associated smell has always been an extremely challenging task. The major limitation in establishing the structure-odor relation is the vague and ambiguous nature of the descriptor-labeling, especially when the sources of odorant molecules are different. With the advent of deep networks, data-driven approaches have been substantiated to achieve more accurate linkages between the chemical structure and its smell. In this study, the deep neural network (DNN) with physicochemical properties and molecular fingerprints (PPMF) and the convolution neural network (CNN) with chemical-structure images (IMG) are developed to predict the smells of chemicals using their SMILES notations. A data set of 5185 chemical compounds with 104 smell percepts was used to develop the multilabel prediction models. The accuracies of smell prediction from DNN + PPMF and CNN + IMG (Xception based) were found to be 97.3 and 98.3%, respectively, when applied on an independent test set of chemicals. The deep learning architecture combining both DNN + PPMF and CNN + IMG prediction models is proposed, which classifies smells and may help understand the generic mechanism underlying the relationship between chemical structure and smell perception.

This approach is interesting but presents limitations. Inter alia, it leads to relatively bad results for odor profiles predictions.

The blog article entitled *"*Digitizing Smell: Using Molecular Maps to Understand Odor" published on September 6th 2022 by Richard C. Gerkin and al., at Google Research teaches how to use graph neural network (GNN) models but nothing more.

The patent document US20220139504 discloses systems and methods for predicting olfactory properties of a molecule. One example method includes obtaining a machine-learned graph neural network trained to predict olfactory properties of molecules based at least in part on chemical structure data associated with the molecules. The method includes obtaining a graph that graphically describes a chemical structure of a selected molecule. The method includes providing the graph as input to the machine-learned graph neural network. The method includes receiving prediction data descriptive of one or more predicted olfactory properties of the selected molecule as an output of the machine-learned graph neural network. The method includes providing the prediction data descriptive of the one or more predicted olfactory properties of the selected molecule as an output. This approach presents limitations.

### Summary

There is disclosed a system comprising: an electronic nose, configured to sense one or more Volatile Organic Compounds in gas phase; one or more machine-learned models configured to receive data as input from said electronic nose and to output multi-class chemical families; wherein one or more machine-learned models have been trained using a labeled training set comprising signatures and annotated chemical families and/or comprising sensorgrams. Developments describe the use of low-dimensional embedding in a chemical space; mapping to another embedding space such as an odor-space; classification; determining vapor pressure, hydrophobicity and/or chemical properties; the use of various sensors including one or more of a humidity sensor, a temperature sensor, a photoionized sensor, or a gas-specific sensor; training by supervised learning and/or semi-supervised learning and/or metric learning and/or online-learning and/or incremental learning and/or transfer learning; the use of attention-based models; implementations occurring inline or in-device, or via remotely accessed computing resources.

There are described systems and methods for chemical family (functional group) detection using an electronic nose using machine learning that overcomes the limitations of existing approaches. The system includes an electronic nose that can sense the presence of gasses or VOCs in a gas environment. An electronic nose is a sophisticated sensing device that emulates the human olfactory system to detect and analyze odors or volatile compounds in the surrounding environment. It consists of an array of chemical sensors that respond to specific odor molecules or volatile compounds. When exposed to an odor, the sensors generate a unique pattern of responses, known as a sensorgram. The sensorgram represents the sensor responses over time, providing a characteristic fingerprint of the volatile compound. This sensorgram data is then processed and analyzed using various algorithms and techniques to identify and classify the odor, quantify its concentration, and distinguish it from other odors.

In the context of an electronic nose, the term "signature" refers to a consolidated representation of the sensorgram data that captures the distinctive characteristics of an odor or volatile compound. The sensorgram, which is a temporal sequence of sensor responses, is transformed or processed to derive a more condensed and informative signature. Most common approach to generate a signature from a sensorgram is by taking the average of the sensor responses over time. This method involves calculating the mean value of each sensor's response across the entire or defined duration of the sensorgram.

The average-based signature provides a compact representation of the overall sensor response pattern over time. It captures the central tendency of the sensorgram data and smooths out any short-term fluctuations or noise. This approach is particularly useful when the sensor responses exhibit consistent trends or when the focus is on the general characteristics of the odor rather than specific temporal dynamics

The electronic nose could be augmented with other sensors such as humidity, temperature, photoionized, or specific gas sensors. The system also includes machine-learned models that receive data from the electronic nose and output multi-class chemical families.

Machine learning models are trained using a labeled training set that contains various types of data, such as signatures, sensorgrams (sensor response patterns), or a combination of both. In addition to the data, the training set is annotated with the corresponding chemical families or even physicochemical properties of volatile organic compounds (VOCs). By incorporating these diverse data sources and annotations, the machine-learned model can learn the complex relationships between the input data and the target labels. This enables the model to accurately classify and identify chemical families based on signatures, sensorgrams, or a combination of both. The integration of annotated chemical families and physicochemical properties further enhances the model's understanding of the underlying chemical characteristics, allowing for more comprehensive and informative predictions.

The method includes transforming the electronic nose signal to a lower dimensional embedding called chemical space. The machine model is trained with metric learning such that nearby data points in the lower dimension represent the same chemical family or physicochemical properties. The lower-dimensional chemical space can be mapped to other embedding spaces such as odor space or can be used for other machine-learning based tasks. The input data is preprocessed by normalization, creating additional features, data augmentations, environmental effect calibration, humidity calibration, drift correction, or normalization by other sensors such as PID.

The system and method can be used to enhance the classification of odor based on chemical families, monitor the chemical family change in the composition in the gas phase, and monitor and detect outliers based on chemical families or low-dimensional embedding. The machine-learning model can be trained with supervised, metric, semi-supervised, or online learning. The machine-learned model can comprise a transformer model, which can improve the accuracy and speed of the system.

A system comprising: an electronic nose, configured to sense gas/presence of one or more Volatile Organic Compounds in gas phase; one or more machine-learned models configured to receive data as input from said electronic nose and to output multi-class chemical families; wherein one or more machine-learned models have been trained using a labeled training set comprising signatures and annotated chemical families and/or comprising sensorgrams.

Some embodiments are associated with various advantages and/or technical effects. In particular, embodiments allow or enable improved or better verifiability, accuracy, efficiency, control, discriminability, security, reusability, and/or safety.

### Brief description of drawings

The present disclosure is illustrated by way of example and not limited to the accompanying figures in which reference numerals indicate similar elements and in which:
FIG. 1 illustrates an embodiment of an electronic nose;
FIG. 2 illustrates different machine learning models according to various embodiments;
FIG. 3 illustrates an overview of relationships between VOCs, signatures, odor space, chemical space and smell.

### Detailed description

### Definitions

### Electronic nose

An electronic nose (e-nose) is a device that is designed to mimic the human sense of smell. It can use an array of chemical sensors to detect and identify different odors or volatile organic compounds (VOCs) in the air.

The electronic nose works by exposing the chemical sensors to a sample of air or gas, which contains the odor or VOCs of interest. The sensors then produce a signal that is analyzed to identify the specific odors or VOCs present in the sample. The output of the electronic nose is typically a pattern or signature of the different odors or VOCs present in the sample. E-nose output typically consists of sensorgrams, which are time-varying patterns representing the response of individual sensors in the sensor array. These sensorgrams provide information about the changes in electrical signals produced by the sensors when exposed to different volatile compounds.

To extract signatures from the sensorgrams, additional data processing techniques are employed. The signatures represent characteristic features or patterns derived from the sensorgram data that are specific to different chemical compounds or chemical families. The sensorgram, which is a temporal sequence of sensor responses, is transformed or processed to derive a more condensed and informative signature. Most common approach to generate a signature from a sensorgram is by taking the average of the sensor responses over time. This method involves calculating the mean value of each sensor's response across the entire or defined duration of the sensorgram.

The average-based signature provides a compact representation of the overall sensor response pattern over time. It captures the central tendency of the sensorgram data and smooths out any short-term fluctuations or noise.

Once the signatures are extracted, they provide a condensed representation of the sensorgram data, highlighting the essential characteristics associated with different chemical compounds or families. These signatures can be used for pattern recognition, classification, or identification tasks. Machine learning algorithms can be trained on the extracted signatures, allowing the electronic nose to recognize and differentiate between different chemical compounds or families based on their sensorgram patterns.

By combining the sensorgram output of an electronic nose with the extraction of signatures, it becomes possible to capture the distinctive features of volatile compounds and enhance the accuracy and reliability of chemical identification and classification.

Electronic noses have a wide range of applications in various fields such as food and beverage industry, environmental monitoring, medical diagnosis, and quality control in manufacturing processes. For example, electronic noses can be used to detect the presence of spoilage or contaminants in food products, to monitor air quality in industrial or urban environments, or to diagnose medical conditions based on breath samples.

### Low-dimensional embedding

Low-dimensional embedding is a method used in machine learning and data analysis to represent high-dimensional data in a lower-dimensional space. It involves mapping data points from their original high-dimensional space to a lower-dimensional space while preserving some of the important characteristics of the data.

There can be several approaches for low-dimensional embedding, including principal component analysis (PCA), t-distributed stochastic neighbor embedding (t-SNE), and uniform manifold approximation and projection (UMAP). These techniques use different mathematical algorithms to preserve different characteristics of the data, such as distances between data points or local structures in the data. Some approaches for metric learning include Learning from relative comparisonswhich is based on the Triplet loss, large margin nearest neighbor, Information theoretic metric learning (ITML).

### Functional group

In organic chemistry, a *functional group* is a substituent or moiety in a molecule that causes the molecule's characteristic chemical reactions. The same functional group will undergo the same or similar chemical reactions regardless of the rest of the molecule's composition. This enables systematic prediction of chemical reactions and behavior of chemical compounds and the design of chemical synthesis. The reactivity of a functional group can be modified by other functional groups nearby. Functional group interconversion can be used in retrosynthetic analysis to plan organic synthesis.

### Multi-class chemical families

In the context of chemical analysis, multi-class chemical families refer to groups of related chemicals that share similar chemical structures or functional groups. These families may include a large number of different compounds, but are typically characterized by a set of common features that allow them to be grouped together based on their chemical properties and behavior.

Multi-class chemical families are often used in analytical chemistry and environmental monitoring to identify and quantify different types of chemicals present in a sample. By analyzing the sample for specific chemical families, rather than individual compounds, it is possible to obtain a more comprehensive picture of the chemical composition of the sample. Examples of multi-class chemical families include polycyclic aromatic hydrocarbons (PAHs), which are a group of organic compounds that contain multiple rings of carbon atoms and are commonly found in combustion products; phenols, which are a class of aromatic compounds that contain a hydroxyl (-OH) group attached to a benzene ring; and alcohols, which are a broad class of organic compounds that contain a hydroxyl group (-OH) attached to a carbon atom.

There is disclosed a system comprising: an electronic nose, configured to sense one or more Volatile Organic Compounds in gas phase; one or more machine-learned models configured to receive data as input from said electronic nose and to output multi-class chemical families; wherein one or more machine-learned models have been trained using a labeled training set comprising signatures and annotated chemical families and/or comprising sensorgrams.

In a development, a signal provided by the electronic nose is converted to low-dimensional embedding in a chemical space.

In a development, a machine model is trained with metric learning such that nearby data-points in the lower dimension represent the same or similar chemical family.

In a development, the low-dimensional chemical space is mapped to another embedding space such as an odor-space.

Mapping chemical space to an odor space involves using chemical information to predict or model the perceived odor of a chemical. This can be achieved using a variety of approaches, including machine learning, statistical analysis, and sensory testing. One approach to mapping chemical space to odor space is to use quantitative structure-activity relationship (QSAR) modeling. QSAR models use mathematical algorithms to relate the chemical structure of a compound to its biological activity or effect, including its odor. Another approach is to use chemical feature analysis to identify the chemical features or descriptors that are most important for predicting odor. This can be done using a variety of statistical methods, such as principal component analysis (PCA) or partial least squares (PLS) regression, to identify the chemical features that are most strongly associated with particular odor qualities. Finally, sensory testing can be used to directly measure the odor of a chemical and map it to a sensory space. This involves presenting a panel of trained human assessors with samples of the chemical and asking them to rate the intensity and quality of its odor. The resulting sensory data can be analyzed using statistical methods such as multidimensional scaling (MDS) or factor analysis to map the odor space.

In a development, the low-dimensional chemical space is further used to classify a chemical family into attributes comprising binary choices such as good smell or bad smell, or into predefined categories.

In a development, the low-dimensional chemical space is further used to perform one or more machine learning-based operations comprising one or more operations selected from the group comprising determining *vapor pressure parameters, hydrophobicity parameters, or chemical properties.* For example, carboxylic acid leads to bad foot smells.

In a development, an electronic nose comprises one or more of a Surface Plasmon Resonance (SPR), a Mach-Zehnder interferometer (MZI), MEMS (Micro-electro-mechanical systems), Capacitive Micromachined Ultrasonic Transducer (CMUT), Aligned Carbon Nanotubes (CNTs), peptides, polymers, piezo resistance mechanisms and/or living cells.

In a development, the electronic nose is augmented with one or more sensors, selected from the group comprising : humidity sensor, temperature sensor, photoionized sensor, or gas-specific sensor.

In a development, handling raw data i.e., without any preprocessing.

In a development, input data is preprocessed by one or more of operations comprising normalizing, generating *additional features or vectors, data augmentation, calibrating humidity, correcting drift, normalizing by other sensors such as PID.*

In a development, the system is further configured to detect and/or monitor one or more changes in the composition in the gas phase.

In a development, the system is further configured to detect and/or monitor one or more outliers based on one or more chemical families and/or low-dimensional embeddings.

In a development, the system is further configured to notify said one or more changes or outliers.

In a development, one or more machine-learning models are trained with supervised learning and/or semi-supervised learning and/or metric learning, and/or online-learning or incremental learning or transfer learning.

In a development, hardware implementations can occur inline or in-device, or via remotely accessed computing resources.

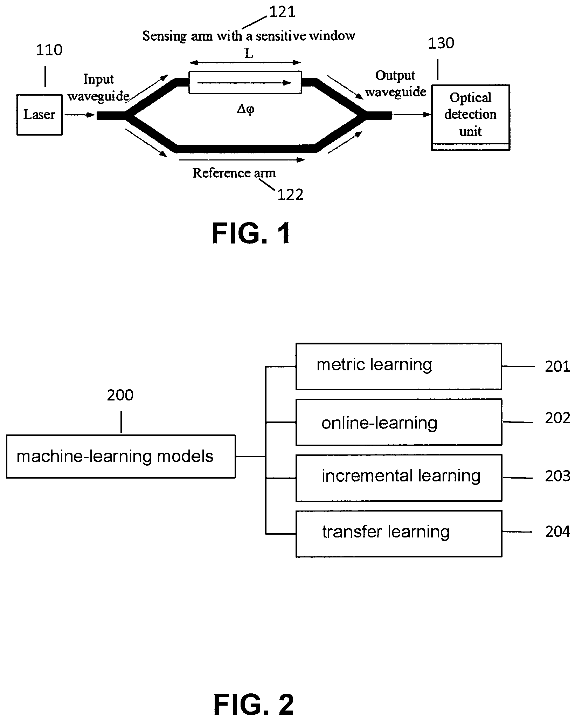

FIG. 1 illustrates an embodiment of an electronic nose.

The structure of an electronic nose can be diverse.

In an embodiment, the "electronic nose" or "analyzing system" comprises a measurement chamber 121 intended to receive the target compounds contained in either gas, liquid or fluid sample, wherein a plurality of separate sensitive sites, each comprising receivers able to interact with the target compounds, is located in said chamber.

Sensors in modern technology are becoming more and more complex. Some of them comprise a Mach-Zehnder Interferometer (MZI). The response signals of the MZI transducer (Mach-Zehnder Interferometer) are signals modulated (by an optical stage) in frequency proportional to the difference in step between the wave in the reference guide and the wave in the measuring guide. This difference allows the reconstruction of an odor sensorgram.

In an electronic nose, VOCs (or fluid sample) are generally injected (passively and/or actively) into a chamber 121 with or without inert gas (e.g., argon). Distinct sensor units (of distinct chemical affinity) forming a "multivariate sensor" in the chamber then react to compounds or VOCs or molecules of the fluid sample and from an analog output, a digital output is determined, and further interpreted.

A "sensorgram" is obtained, comprising substantially parallel curves, each curve being the response of one sensor unit to compounds or VOCs. Amplifiers can be used. From said sensorgram, a signature is determined (such as a radar chart), said signature being associated with one or more predefined "odors" (interpretation of the presence of compounds can be post-processed).

Databases can be built, in particular in the form of signatures and/or low-dimensional embeddings, i.e., using metric learning, PCA or trained neural networks.

In an embodiment, the sensor comprises an optical integrated circuit comprising: a lower layer called substrate; a first coupling means adapted to couple a radiation incident light (laser) 100 to the integrated optical circuit; a directional splitter connected to the first coupling means and configured to separate the light radiation coupled by the first means from the second means coupling to at least one pair of waveguides comprising: a first waveguide known as a sensitive arm 121 in which is propagated a first portion of the light radiation, said sensitive arm 121 being exposed to a first ambient medium and at least one compound to be detected that induces a modification of the local refractive index perceived by the evanescent part of the electromagnetic field of the first portion of the light radiation, and a second waveguide called a reference arm 122 in which is propagated a second portion of the light radiation, an encapsulation layer encapsulating the reference arm, said layer being impermeable to the compound(s) to be detected, so as to be able to detect that the reference arm is exposed only to a second medium the same nature as the first ambient environment, and devoid of said compound to be detected ; a directional combiner combining the first portion of the radiation from said reference arm, called the first transmitted portion, and the second portion of the light radiation from said sensitive arm, called the "sensitive arm" second transmitted portion, to form a transmitted radiation ; second coupling means adapted to couple said radiation; and transmitted to a medium external to the integrated optical circuit; an upper layer called superstrate covering at least the first and the second layers of the optical integrated circuit; second coupling means, the directional separator and the combiner directional and does not cover the sensitive and reference arm, said encapsulation layer being deposited on top of the superstrate.

In an embodiment, the sensor comprises: a laser source 100 configured to emit incident light radiation; an integrated optical circuit according; an optical detection system 130 adapted to detect a light radiation; - an integrated optical circuit from the second coupling means and generate a signal representative of the evolution over time of the detected light intensity; a unit for processing the said signal, adapted to determine, from the intensity detected, the evolution over time of the phase shift between the first portion and the second portion transmitted and the second portion transmitted.

Sensors in modern technology are becoming more and more complex. In some embodiments, an electronic nose can comprise one or more of SPR, MZI, MEMS, CMUT, and/or Carbon Nanotubes.

### Surface Plasmon Resonance (SPR)

Surface plasmon resonance (SPR) occurs where electrons in a thin metal sheet become excited by light that is directed to the sheet with a particular angle of incidence, and then travel parallel to the sheet. Assuming a constant light source wavelength and that the metal sheet is thin, the angle of incidence that triggers SPR is related to the refractive index of the material and even a small change in the refractive index will cause SPR to not be observed. This makes SPR a possible technique for detecting particular substances (analytes) and SPR biosensors have been developed to detect various important biomarkers

### Mach-Zehnder interferometer (MZI)

Response signals of a MZI transducer (Mach-Zehnder Interferometer) are signals modulated (by an optical stage) in frequency proportional to the difference in step between the wave in the reference guide and the wave in the measuring guide. This difference allows the reconstruction of an odor sensorgram. In variants, the multivariate sensor comprises a Fizeau interferometer or a Fabry-Pérot interferometer or a Jamin interferometer or a Ramsey-Bordé interferometer.

### MEMS

MEMS (Micro-electro-mechanical systems) incorporate both electronic and moving parts. MEMS are made up of components between 1 and 100 micrometers in size, and MEMS devices generally range in size from 20 micrometers to a millimeter, although components arranged in arrays (e.g., digital micromirror devices) can be more than 1000 mm2. They usually consist of a central unit that processes data (an integrated circuit chip such as microprocessor) and several components that interact with the surroundings (such as microsensors)

### Capacitive Micromachined Ultrasonic Transducer, acronym CMUT

In an embodiment, the multivariate sensor according to some embodiments comprises a Capacitive Micromachined Ultrasonic Transducer, acronym CMUT. CMUTs are the transducers where the energy transduction is due to change in capacitance. CMUTs are constructed on silicon using micromachining techniques. A cavity is formed in a silicon substrate, and a thin layer suspended on the top of the cavity serves as a membrane on which a metallized layer acts as an electrode, together with the silicon substrate which serves as a bottom electrode. In an embodiment, the multivariate sensor can comprise a large number of CMUTs. In particular, CMUT-on-CMOS technology along with a flip-chip process can allow integration of CMUTs with front-end electronics.

### Carbon Nanotubes

Aligned Carbon Nanotubes (CNTs) can be grown at low temperature (250 °C) using Plasma Enhanced Chemical Vapor Deposition (PECVD). CNTs can then be used to identify VOCs in an improved sensitivity condition to gas at room temperature. Sensitivity and stability of carbon nanotube sensors can be improved via growing Titanium Dioxide Nanowires (TiO2-NW) on the surface of CNTs through a hydrothermal method. A threefold increase in sensitivity and a 30-second decrease in response time can be observed as a result of the CNT-TiO2 sensor compared to a pristine CNT sensor.

### Other sensor embodiments

In an advantageous embodiment, peptides are used. Peptides are short chains of between two and fifty amino acids, linked by peptide bonds. Chains of fewer than ten or fifteen amino acids are called oligopeptides, and include dipeptides, tripeptides, and tetrapeptides. A "polypeptide" is a longer, continuous, unbranched peptide chain of up to approximately fifty amino acids. Hence, peptides fall under the broad chemical classes of "biological polymers and oligomers", alongside nucleic acids, oligosaccharides, polysaccharides, and others.

In some embodiments of the invention, polymers (on silicon or derivative materials like SiO2 or other traditional materials in micromachining MEMS or silicon photonics) can be used. Silicon is a chemical element with the symbol Si and atomic number 14. It is a semiconductor.

In some embodiments, piezo resistance mechanisms can be used. In some embodiments, living cells can be used. In some embodiments, combinations of the above means can be used, e.g., a combination of peptides and polymers. Depending on use cases (i.e., general purpose or specific detection), the number and/or types of sensor units are diverse.

FIG. 2 illustrates different machine learning models according to various embodiments.

Machine learning models 200 can comprise one or more of: metric learning 201, online-learning 202, incremental learning 203 and/or transfer learning 204.

### Metric learning

Similarity learning is closely related to distance metric learning. *Metric learning* is the task of learning a distance function over objects. A metric or distance function obeys four properties: non-negativity, identity of indiscernibles, symmetry and subadditivity (or the triangle inequality). In practice, metric learning algorithms ignore the condition of identity of indiscernibles and learn a pseudo-metric.

*Supervised learning* is a type of machine learning in which an algorithm learns to map inputs to outputs based on a labeled dataset. The labeled dataset consists of input/output pairs, where the inputs are the features and the outputs are the labels. The goal of supervised learning is to train a model to accurately predict the output for new inputs that it has not seen before. During the training process, the algorithm uses the labeled data to learn the relationship between the input and output variables. This typically involves adjusting the model's parameters to minimize the difference between the predicted output and the actual output in the labeled data. Once the model is trained, it can be used to make predictions on new, unseen data. The performance of the model is typically evaluated on a separate set of data called the validation set or test set.

*Semi-supervised* learning is a type of machine learning that combines both labeled and unlabeled data to improve the accuracy of a model. In semi-supervised learning, only a subset of the data is labeled, while the rest of the data is unlabeled.

*Metric learning* 201 is a type of machine learning that focuses on learning a distance metric between data points. The goal of metric learning is to learn a function that can measure the similarity or dissimilarity between data points in a meaningful way, so that similar data points are closer together in the learned metric space and dissimilar data points are further apart. In metric learning, the distance metric is learned from the data itself, rather than being predefined. This means that the distance metric can be tailored to the specific characteristics of the data and the task at hand. There are several approaches to metric learning, including Siamese networks, triplet networks, and contrastive loss functions. These approaches typically involve training a neural network to learn a mapping from the input data to a learned metric space, where the distance between data points corresponds to their similarity. The network is trained by minimizing a loss function that encourages similar data points to be closer together in the learned metric space and dissimilar data points to be further apart.

Online learning 202 is a type of machine learning in which the model is updated continuously as new data becomes available, rather than being trained on a fixed dataset. In online learning, the model learns from data as it arrives in a stream, and can adapt to changes in the data distribution over time. Online learning algorithms are typically designed to be computationally efficient and scalable, allowing them to handle large and constantly changing data streams. Online learning typically involves adjusting the model's parameters based on the new data, using techniques such as stochastic gradient descent or online convex optimization.

*Incremental learning* 203 is a type of machine learning in which the model is trained on new data incrementally over time, rather than being trained on a fixed dataset. Incremental learning is similar to online learning, but instead of learning from a continuous stream of data, it learns from multiple, discrete batches of data. Each batch of data is used to update the model, and the model is then used to make predictions on new, unseen data. Incremental learning algorithms typically involve a combination of techniques from batch learning and online learning. For example, the model parameters may be initialized using a pre-trained model, and then updated incrementally as new data becomes available using techniques such as stochastic gradient descent or online convex optimization.

*Transfer learning* 204 is a technique in machine learning where a pre-trained model developed for one task is used as a starting point for a new, related task. A model trained on a large and diverse dataset can learn general features that are useful across multiple tasks, and these features can be transferred to new, related tasks to improve performance and reduce training time. By leveraging a pre-trained model, transfer learning can often achieve higher accuracy with less data than training a new model from scratch. There are several ways to apply transfer learning, depending on the similarity between the source and target tasks. In some cases, the entire pre-trained model is fine-tuned on the new task, while in other cases, only the last few layers of the model are retrained on the new task.

In a development, a machine-learned model comprises an attention-based model.

An attention-based model is a type of machine learning model that focuses on learning to weigh different inputs or parts of the input data differently, depending on their relevance to the task at hand. In a traditional machine learning model, all inputs are treated equally. In an attention-based model, the model learns to assign different weights to different inputs, depending on their relevance to the considered task. Attention-based models can use a variety of neural network architectures, such as recurrent neural networks (RNNs), convolutional neural networks (CNNs), or transformer networks. The attention mechanism can be trained jointly with the rest of the model, using backpropagation and gradient descent.

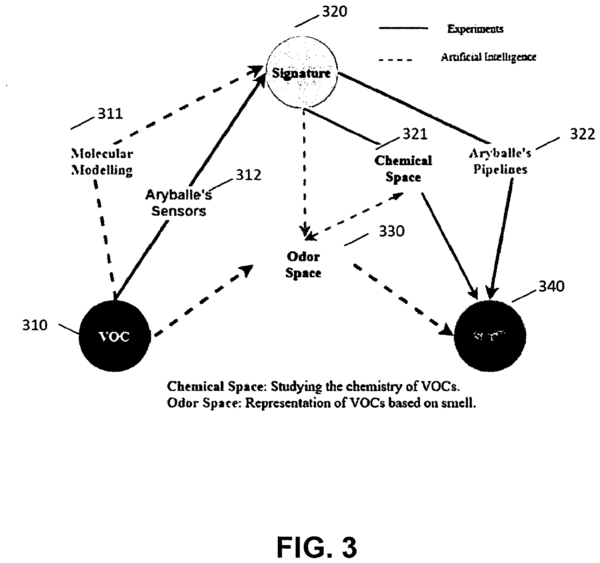

FIG. 3 illustrates an overview of relationships between VOCs, signatures, odor space, chemical space and smell.

VOC 310 designates Volatile Organic Compounds. VOCs are represented as SMILES in theoretical and computational chemistry. These smiles are then used to identify the structure-to-activity relationship also known as QSAR (Quantitative Structural Activity Relationship). In some embodiments, the activity can be the smell associated with a VOC.

Latent low-dimensional space can show a fuzzy relationship between SMILES and smell is known as Odor-space 930.

Most theoretical work is designed for determining relationships between VOC 310, Odor Space 330 and smell 340

In reality, most often, emitted VOCs 310 are not known (so as the composition of VOCs in the gas phase). For example, VOCs are released by wine or perfume. In general, we do not know these VOCs.

Instruments such as electronic nose convert emitting VOC (or mixture of VOCs) into a numerical signal. We refer to this numerical signal as a Signature 320.

Sensors 312 are biological-based multivariate sensors that convert the binding of VOCs with peptide to numerical data using optical methods. For a VOC, the ranking of sensors is directly associated with binding strength.

Molecular modeling 311 approaches such as docking and molecular dynamics can be used to estimate the binding energy of a VOC with a given peptide sensor. Drawbacks comprise the fact that all the biological molecular modeling simulation is validated in the liquid phase and the electronic nose works in the gas phase.

Signatures to smell 322 according to some embodiments can determine a mapping to convert signatures to smell.

The path 321 (Signature-Chemical Space-Odor) can require a plurality of processes: a) mapping signature to Chemical functional group (example: alcohol, aldehyde, ketone etc.), and mapping Chemical space to odor. It is also possible to map Chemical-space to Odor-space.

There is described a computer-implemented method comprising the steps of: representing a plurality of pure Volatile Organic Compounds as a plurality of simplified molecular-input line-entry system values ; converting said plurality of simplified molecular-input line-entry system values into one or more graphs comprising nodes and edges; determining a signature (binding affinity, for example represented as a radar chart) of a pure VOC by using a probabilistic model that predicts odor profile of a pure VOC. In a development, the probabilistic model is trained by using an attention-based model. In a development, the attention-based model comprises one or more Graph Neural Networks (Graph convolutional network, Graph attention network, Gated graph sequence neural network). In a development, the method further comprises predicting signatures of individual VOC (ex: emitting VOCs, alcohol, smell lavender) based on vapor pressure.; one of the VOC can be humidity/water. In a development, the method further comprises predicting signatures of mixtures weighted based on individual VOC (a+b+c) based on vapor pressure. The weight can be based on vapor pressure (property of VOC cannot be changed) and/or dilution / concentration of vapor pressure. In a development, humidity is an external environmental factor, controllable (in a lab condition) but is otherwise non controllable (like temperature). The signature of water designates humidity. Conditions can be geographically determined. In a development, affine transformations with normalized vapor pressures of VOCs can be used. In a development, the method further comprises determining odor profiles associated with mixtures of pure VOCs based on said signatures, by taking into account different dilution or concentration levels. In a perspective, some embodiments constitute a "data augmentation technique" going from one molecule to a mixture of different molecules in different concentrations. So, with one VOC, it is possible to generate more data at different a) concentration levels and of b) humidity levels
The disclosed methods can take the form of an entirely hardware embodiment (e.g., FPGA), an entirely software embodiment or an embodiment containing both hardware and software elements. Software embodiments include but are not limited to firmware, resident software, microcode, etc. The invention also can take the form of a computer program product accessible from a computer-usable or computer-readable medium providing program code for use by or in connection with a computer or any instruction execution system. A computer-usable or computer-readable can be any apparatus that can contain, store, communicate, propagate, or transport the program for use by or in connection with the instruction execution system, apparatus, or device. The medium can be an electronic, magnetic, optical, electromagnetic, and/or semiconductor system (or apparatus or device) or a propagation medium or the like.

There is disclosed a computer program comprising instructions for carrying out one or more of the steps of the method when said computer program is executed on a computer device.

## Claims

**1.** A system comprising:
- an electronic nose, configured to sense one or more Volatile Organic Compounds in gas phase;
- one or more machine-learned models configured to receive data as input from said electronic nose and to output multi-class chemical families;
- wherein one or more machine-learned models have been trained using a labeled training set comprising signatures and annotated chemical families and/or comprising sensorgrams.

**2.** The system of claim 1; wherein machine learning model is trained with sensorgram and/or signatures.

**3.** The system as described in claim 1 and 2 is further **characterized by** the training of a machine learning model using extracted features from sensorgrams and/or signatures..

**2.** The system of Claim 1, wherein a signal provided by the electronic nose is converted to low-dimensional embedding in a chemical space.

**3.** The system of Claim 2, wherein a machine model is trained with metric learning such that nearby data-points in the lower dimension represent the same or similar chemical family.

**4.** The system of Claim 2, wherein the low-dimensional chemical space is mapped to another embedding space such as an odor-space.

**5.** The system of Claim 4, wherein the low-dimensional chemical space is further used to build a machine learning model such as to classify a chemical family into attributes comprising binary choices such as good smell or bad smell, or into predefined categories.

**6.** The system of Claim 4, wherein the low-dimensional chemical space is further used to perform one or more machine learning-based operations comprising one or more operations selected from the group comprising determining vapor pressure parameters, hydrophobicity parameters, or chemical properties.

**7.** The system of any preceding Claim, wherein electronic nose comprises one or more of: a Surface Plasmon Resonance, a Mach-Zehnder interferometer, Micro-electro-mechanical systems, a Capacitive Micromachined Ultrasonic Transducer , Aligned Carbon Nanotubes, peptides, polymers, piezo resistance mechanisms and/or living cells.

**8.** The system of any preceding Claim, wherein the electronic nose is augmented with one or more sensors, selected from the group comprising : humidity sensor, temperature sensor, photoionized sensor, or gas-specific sensor. 8.

**8.** The system of any preceding Claim, handling raw data i.e., without any preprocessing.

**9.** The system of any preceding Claim, wherein input data is preprocessed by one or more of operations comprising normalizing, generating *additional features or vectors, data augmentation, calibrating humidity, correcting drift, normalizing by other sensors such as PID.*

**10.** The system of any preceding claim, further configured to detect and/or monitor one or more changes in the composition in the gas phase.

**11.** The system of any preceding claim, further configured to detect and/or monitor one or more outliers based on one or more chemical families and/or low-dimensional embeddings.

**12.** The system of claims 10 or 11, further configured to notify said one or more changes or outliers.

**13.** The system of any preceding claim, wherein one or more machine-learning models are trained with supervised learning and/or semi-supervised learning and/or metric learning, and/or online-learning or incremental learning or transfer learning.

**14.** The system of any preceding claim, wherein a machine-learned model comprises an attention-based model.

**15.** The system of any preceding claim, wherein hardware implementations can occur inline or in-device, or via remotely accessed computing resources.
